# EUROPÄISCHE PATENTANMELDUNG

(11) **EP 0 966 938 A2**
(43) Veröffentlichungstag der Anmeldung: **29.12.1999**
(21) Anmeldenummer: 99250204.7
(22) Anmeldetag: 23.06.1999
(51) Int. Cl.: A61F 7/02, A61H 33/06

(54) **Keramik-Diffusionsstuhl (KED)**

(30) Priorität: 24.06.1998 DE 19982011927U; 20.07.1998 DE 19981033788
(71) Anmelder: Pannach, Jürgen, 12305 Berlin (DE)
(72) Erfinder: Pannach, Jürgen, 12305 Berlin (DE)

(57) **Zusammenfassung**

Die Wirungsweise des **Keramik- Diffusionsstuhles zur Dampfbehandlung (KEDD)** besteht darin, daß durch feinste Kapillarröhren im speziell behandelten Ton (Keramik), unmerklich Dämpfe der Behandlungsflüssigkeit austreten und über die Haut in die Gefäße (Gelenke) eindringen.

Der **Keramik- Diffusionsstuhl zur Dampfbehandlung** dient somit der Therapie verschiedenster Erkrankungen (z.B.- rheumatischer). Die Anwendung erfolgt über einen langen Zeitraum. Der **KEDD** kann in ansprechender Gestaltung Bestandteil der Wohnungseinrichtung sein. Der Benutzer wird dadurch in die Lage versetzt, dessen Wirkung auch ohne momentane Beschwerden aufzunehmen, um sein Leiden zu lindern.

Der Patient stellt über einen längeren Zeitraum durch Eigentest's den Typ der Behandlungflüssigkeit fest, ebenso die angenehmste Temperatur und damit die Dampfmenge. Somit nimmt er direkten Einfluß auf seinen Behandlungserfolg (psychische Stimulanz). Alle ermittelten Werte werden im *programmierbaren Steuergerät (PSG)* gespeichert und können in der selbst ermittelten- bekömmlichsten Kombination durch Knopfdruck jederzeit abgefordert werden.

## Beschreibung

**Der Keramik- Diffusionsstuhl zur Dampfbehandlung** (KEDD) dient der Therapie von z.B. rheumatischen Erkrankungen. Die Wirkungsweise besteht darin, daß die angenehme Wärme des natürlichen Tones, verbunden mit dem wohl dosierten Eindringen verschiedenster idividuell zusammenstellbarer Behandlungsflüssigkeiten über die Haut in das Gewebe und in die Gelenk, es zu einer *spürbaren* und *anhaltenden* Minderung der Schmerzen kommt.

Über einen stufenlosen Regler kann sich der Patient die jeweilige Behandlungstemperatur selbständig einstellen oder nach erfolgter individueller Datenerfassung (angnehmste Temperatur und Feuchte; Schmerzgrad; Behandlungsflüssigkeitstyp usw.), durch das Steuergerät (PSG) automatisch abfordern.

Die Fertigung der Stühle erfolgt in bestimmten Grundgrößen oder auf Wunsch nach individuell abgenommenen Körpermaßen. Desweiteren kann der **KEDD** fahrbar- auch mit Elektroantriebgefertigt werden.

Zur effektiveren Verwertung der Behandlungsflüssigkeit kann ein spezieller Umhang mitgeliefert werden.

Eine *Umkehrung* des beschriebenen Wärmebetriebes in eine Kältebehandlung ist durch den Einbau dementsprechender Aggregate möglich.

### Schutzansprüche

**1.** Der **Keramik- Diffusionsstuhl zur Dampfbehandlung (KEDD)** besteht aus gebranntem Ton in Verbindung mit einem Trägergestell und ist durchdrungen von feinsten Kapillaröffnungen (1) verteilt über die gesamte Sitz (2)- und Lehnfläche (3)- oder auch erweitert auf Arm-, Nacken- und Beinbereiche bzw. auf den gesamten Körper,
   *dadurch gekennzeichnet,*
   - daß diese Kapillaröffnungen (1) in Hohlräume (4) münden, die die Sitz- und Lehnflächen durchziehen und
   - dem Austreten von verdampfter Flüssigkeit dienen, wobei in den
   - unteren Flüssigkeitstank (5) über den Einfüllstutzen (6)
   - die Behandlungsflüssigkeit (7) eingefüllt wird und nach Bedarf durch den Entlerugsstutzen(8) abgelassen werden kann,
   - die verschließbare Öffnung (9) dient der Entlüftung während des Austauschens der Flüssigkeit.
**2. Der Keramik- Diffusiongstuhl zur Dampfbehandlung** nach Schutzanspruch 1
   *dadurch gekennzeichnet,*
   - daß in den Sitzbereich (2) die elektrische Heizspirale (10), in den Lehnbereich (3) die Heizspirale (11) und in den Flüssigkeitstank (5) die Heizspirale (12) eingearbeitet ist,
   - eine Ansteuerung (Temperatur) der Heizspiralen 10,11 und 12 im Zusammenwirken mit den gewonnenen Parametern, der am Patienten befestigten Temperatur- und Feuchtefühlern (13) erfolgen kann und
   - diese Parameter im programmierbaren Steuergerät- PSG (14) zusammengefaßt und für den Automatikbetrieb verarbeitet werden.
   - Handbetrieb ist über den Hand- Automatikumschalter (15) möglich, wobei die
   - stufenlose Temperatureinstellung über die jeweiligen Taster (16,17,18) erfolgt und an der
   - Kombi- Temperaturanzeigeeinheit (19) ablesbar ist.

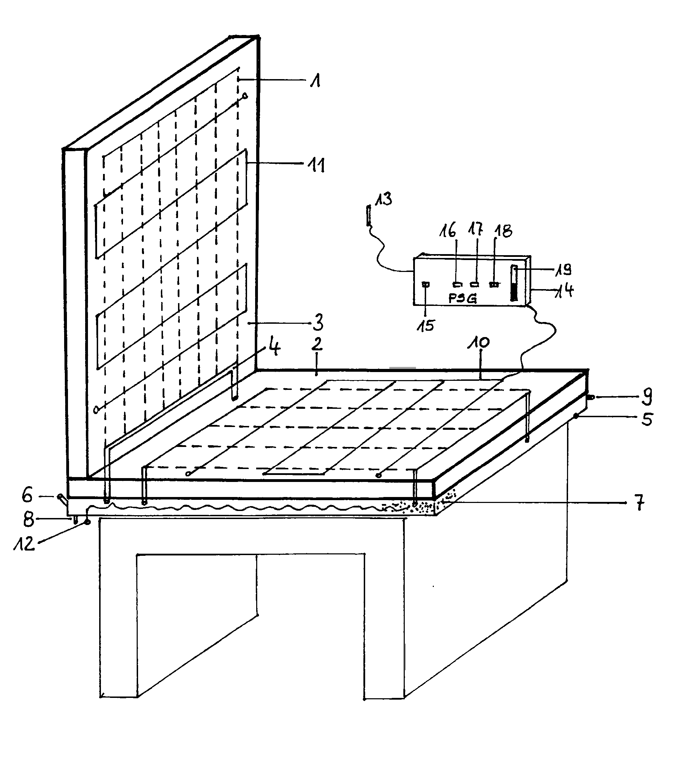

## Patentansprüche

**2. 1.1. Der Keramik- Diffusionastuhl zur Dampfbehandlung (KEDD**) besteht aus gebranntem Ton in Verbindung mit einem Trägergestell und ist durchdrungen von feinsten Kapillaröffnungen (1) verteilt über die gesamte Sitz (2)- und Lehnfläche (3)- oder auch erweitert auf Arm-, Nacken- und Beinbereiche bzw. auf den gesamten Körper, ***dadurch gekennzeichnet,***
- **daß** diese Kapillaröffnungen (1) in Hohlräume (4) münden, die die Sitz- und Lehnflächen durchziehen und
- dem Austreten von verdampfter Flüssigkeit dienen, wobei in den
- unteren Flüssigkeitstank (5) über den Einfüllstutzen (6)
- die Behandlungsflüssigkeit (7) eingefüllt wird und nach Bedarf durch den Entleerungsstutzen (8) abgelassen werden kann,
- die verschließbare Öffnung (9) dient der Entlüftung während des Austauschens der Flüssigkeit

**2. 1.2.** Der **Keramik- Diffusionsstuhl zur Dampfbehandlung** nach Patentanspruch 2.1.1. ***dadurch gekennzeichnet,***
- **daß** in den Sitzbereich (2) die elektrische Heizspirale (10), in den Lehnbereich (3) die Heizspirale (11) und in den Flüssigkeitstank (5) die Heizspirale (12) eigearbeitet ist,
- eine Ansteuerung (Temperatur) der Heizspirale 10, 11 und 12 im Zusammenwirken mit den gewonnenen Parametern, der am Patientenbefestigten Temperatur- und Feuchtefühlern (13) erfolgen kann und
- diese Parameter im programmierbaren Steuergerät- PSG (14) zusammengefaßt und für den Automatikbetrieb verarbeitet werden.
- Handbetrieb ist über den Hand- Automatikumschalter (15) möglich, wobei die
- stufenlose Temperatureinstellung über die jeweiligen Taster (16,17,18) erfolgt und an der
- Kombi- Temperaturanzeigeeinheit (19) ablesbar ist.

**2. 2. Beschreibung**
De**r Keramik- Diffussionsstuhl zur Dampfbehandlung (KEDD)** dient der Therapie von z.B. rheumatischen Erkrankungen. Die Wirkungsweise besteht darin, daß die angenehme Wärme des natürlichen Tones (mit feinsten Kapillaröffnungen), *verbunden* mit dem wohldosierten Eindringen verschiedenster *individuell zusammenstellbarer* Behandlungsflüssigkeiten durch die Kapillaröffnungen über die Haut in das Gewebe und in die Gelenke, es zu einer spürbaren und anhaltenden Minderung der Schmerzen kommt.
Über einen stufenlosen Regler kann sich der Patient die jeweilige Behandlungstemperatur selbständig einstellen oder nach erfolgter individueller Datenerfassung (angenehmste Temperatur und Feuchte; Schmerzgrad; Behandlungsflüssigkeitstyp usw.), über das Steuergerät (PSG) automatisch abfordern.
Die Fertigung der Stühle bzw. Körpersegmente für Arm, Bein, Nacken usw., erfolgt in bestimmten Grundgrößen oder auf Wunsch nach individuell abgenommenen Körpermaßen. Desweitern kann der **KEDD** fahrbar (auch mit Elektroantrieb) gefertigt werden.
Zur effektiveren Verwertung der Behandlungsflüssigkeit kann ein spezieller Umhang mitgeliefert werden.
Eine Umkehrung des beschriebenen Wärmebertiebes in eine Kältebehandlung, ist durch den Einbau dementsprechender Aggregate möglich.
